# EUROPEAN PATENT APPLICATION

(11) **EP 4 641 197 A2**
(43) Date of publication of application: **29.10.2025**
(21) Application number: 25196483.9
(22) Date of filing: 18.05.2021
(51) Int. Cl.: G01N 33/53

(54) **INJECTION WATER PRODUCTION FACILITY, METHOD OF PRODUCING INJECTION WATER**

(30) Priority: 30.06.2020 JP 2020112719
(62) Divisional of application: 21834053.7
(71) Applicant: Nomura Micro Science Co., Ltd., Atsugi-shi, Kanagawa 243-0021 (JP); Sophia School Corporation, Tokyo 102-8554 (JP)
(72) Inventor: KIMOTO, Hiroshi, Atsugi-Shi, Kanagawa 243-0021 (JP); IIYAMA, Masamitsu, Atsugi-Shi, Kanagawa 243-0021 (JP); HASHIMOTO, Takeshi, Chiyoda-ku, Tokyo 102-8554 (JP); HAYASHITA, Takashi, Chiyoda-ku, Tokyo 102-8554 (JP)
(74) Representative: DREISS Patentanwälte PartG mbB

(57) **Abstract**

An injection water production facility (800), said injection water production facility (800) comprising an endotoxin detection device (100) having a supply unit (300) configured to supply a fluorescent substance (10) to a sample (960), the fluorescent substance (10) having a structure in which a fluorescent site (20) and a recognition site (40) that recognizes a specific site (51) of a molecular structure of an endotoxin (50) are connected by a spacer (30).

## Description

### Technical Field

The present disclosure relates to a method of detecting an endotoxin in a test subject sample and an endotoxin detection device, and further relates to a purified water production facility, an injection water production facility, a method of producing purified water and a method of producing injection water.

### Background Art

Purified water is obtained by purifying normal water by distillation, ion exchange using an ion exchange resin tower, an electric desalting device (EDI), or the like, reverse osmosis or ultrafiltration, an ultraviolet irradiation device (UV), or any combination thereof, and is used not only as a raw material of a formulation but also for preparation of a reagent and the like. Sterilized purified water obtained by sterilizing the purified water cannot be used for production of injection water (WFI, Water for Injection) used for production of an injection solution without testing for the presence of a pyrogen (an endotoxin). The WFI is adapted to a predetermined endotoxin test after sterilizing purified water.

As an example of a WFI production facility, there is a technique described in Japanese Patent Application Laid-Open (JP-A) No. 2019-025456. For the production of WFI, it is essential to highly remove endotoxins from purified water produced in a purified water production facility by means of an endotoxin removal facility such as a distiller or a polymer membrane filtration device (preferably, an ultrafiltration membrane device).

An endotoxin is a lipopolysaccharide that is a cell wall component of gram-negative bacteria, and is a representative pyrogen ubiquitously distributed in a living environment. When an endotoxin enters blood, actions such as fever, septic shock, multiple organ failure, and tachycardia occur. Therefore, strict management is required in the manufacture of pharmaceutical products and medical equipment, particularly liquids directly introduced into a living body and medical equipment such as pharmaceutical water, syringes, artificial organs, and dialysis membranes. For example, in the management standard for injection water in the "Japanese Pharmacopoeia (JP17) Quality Conformance Test", endotoxins are regulated to less than 0.25 EU/mL.

Detection of endotoxins has been performed by a Limulus test utilizing the coagulation of blood cell components of horseshoe crabs by endotoxins. In the Limulus test, it is currently standard to perform the test using a lysate reagent extracted from the blood of a horseshoe crab, and it takes 1 to 2 hours to obtain the test result. Although an online measurement device using the Limulus test has also been devised, it is difficult to obtain a practical quantitative lower limit, and quantitativity and reproducibility are poor. For example, since the reagent itself is biologically derived, its performance may vary depending on the batch of reagents. For this reason, a detection method capable of obtaining test results more quickly and performing online measurement with practical accuracy has been desired. In order to obtain a lysate reagent, it is necessary to capture a wild organism such as a horseshoe crab and collect blood. Therefore, from the viewpoint of animal welfare, too, development of an alternative means that does not use the blood of a horseshoe crab has been desired.

For example, as disclosed in Japanese Patent Application Laid-Open (JP-A) No. 2016-151482 and Japanese Patent Application Laid-Open (JP-A) No. 2007-093378, detection of endotoxins by an electrochemical method has also been attempted. However, the detection of endotoxins by an electrochemical method has many problems such as the manufacturing technique or mass productivity of an electrode used for detection, the required maintenance frequency, or measurement accuracy and rapidity, particularly in order to obtain a practical quantitative lower limit, and this method has not been put into practical use.

### SUMMARY OF INVENTION

### Technical Problem

When an abnormality occurs in the endotoxin removal capacity of an endotoxin removal facility, the endotoxin concentration in WFI easily increases, which is unsuitable for production of an injection solution. Particularly, in recent years, in order to reduce energy consumption, there is an increasing tendency to employ a polymer membrane filtration device instead of a conventional distiller as such a facility. However, since a resin material is used as a polymer membrane in a polymer membrane filtration device, there are concerns that an abnormality is more likely to occur than in a distiller since, for example, the resin material is damaged each time heat sterilization is performed. These concerns have hindered the adoption of polymer membrane filtration devices in endotoxin removal facilities.

Therefore, in order to stably produce WFI of the required water quality, it is necessary to perform maintenance such as immediately replacing or repairing a component such as a polymer membrane when an abnormality occurs in an endotoxin removal facility. However, a method capable of rapidly detecting an abnormality in endotoxin removal capacity has not yet been realized.

An object of the present disclosure is to provide a method of detecting an endotoxin, an endotoxin detection device, a purified water production facility, an injection water production facility, a method of producing purified water and a method of producing injection water that can be easily and quickly quantified even at a low concentration, without utilizing a Limulus test or an electrochemical method.

### Solution to Problem

A method of detecting an endotoxin of the present disclosure is a method comprising detecting an endotoxin from a test subject sample using a fluorescent substance having a structure in which a fluorescent site and a recognition site are connected by a spacer, wherein the recognition site recognizes a specific site of a molecular structure of an endotoxin.

Here, it is desirable that the spacer in the fluorescent substance has from 1 to 10 carbon atoms, and each chemical bond of a straight chain connecting the fluorescent site to the recognition site via the spacer is only a single bond. In this case, atoms other than carbon such as nitrogen, oxygen, and sulfur may be interposed in the middle of the straight chain, or there may be a branch from the middle of the straight chain.

The fluorescent substance may be dpa-HCC represented by the following Formula (1), which has a dipicolylamino group to which a metal ion Mⁿ⁺, wherein n is a natural number, is coordinated as the recognition site, and has 7-hydroxycoumarin-3-carboxylic acid as the fluorescent site, and wherein the spacer has one carbon atom. In this case, the specific site is a phosphate group of an endotoxin.

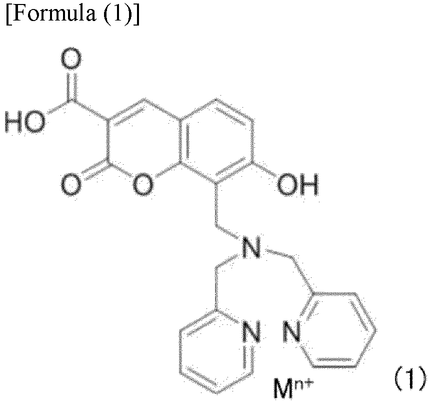

The metal ion in Formula (1) above is desirably a copper ion (Cu²⁺), a nickel ion (Ni²⁺), or a cobalt ion (Co²⁺), and among these, a copper ion is most desirable.

The fluorescent substance may be C1-APB represented by the following Formula (2), which has phenylboronic acid as the recognition site, and has pyrene as the fluorescent site, and wherein the spacer has an amide bond. In this case, the specific site is a sugar chain moiety of an endotoxin.

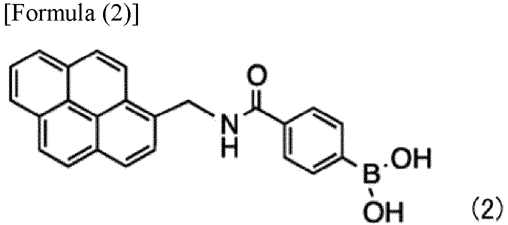

An endotoxin detection device of the disclosure includes a sample introduction unit into which a test subject sample is introduced; a supply unit configured to supply a fluorescent substance to the sample; the fluorescent substance having a structure in which a fluorescent site and a recognition site that recognizes a specific site of a molecular structure of an endotoxin are connected by a spacer and a reaction unit in which the sample and the fluorescent substance react with each other; a detection unit configured to detect light emission or color development (hereinafter, they are collectively referred to as "light emission") of the fluorescent substance subjected to the reaction.

Here, it is desirable that the spacer in the fluorescent substance has from 1 to 10 carbon atoms, and each chemical bond of a straight chain connecting the fluorescent site to the recognition site via the spacer is only a single bond. In this case, atoms other than carbon such as nitrogen, oxygen, and sulfur may be interposed in the middle of the straight chain, or there may be a side chain from the middle of the straight chain.

The fluorescent substance may be the dpa-HCC represented by Formula (1), which has a dipicolylamino group to which a metal ion Mⁿ⁺, wherein n is a natural number, is coordinated as the recognition site, and has 7-hydroxycoumarin-3-carboxylic acid as the fluorescent site, and wherein the spacer has one carbon atom. In this case, the specific site is a phosphate group of an endotoxin. The metal ion in Formula (1) is desirably a copper ion (Cu²⁺), a nickel ion (Ni²⁺), or a cobalt ion (Co²⁺), and among these, a copper ion is most desirable.

The fluorescent substance may be the C1-APB represented by Formula (2), which has phenylboronic acid as the recognition site, and has pyrene as the fluorescent site, and wherein the spacer has an amide bond. In this case, the specific site is a sugar chain moiety of an endotoxin.

The sample may be collected from purified water produced in a purified water production facility or injection water produced in an injection water production facility.

A purified water production facility of the disclosure includes: a purified water production unit that obtains purified water from raw water via at least one of a reverse osmosis membrane device that performs reverse osmosis filtration of the raw water or an ion exchange device that performs ion exchange of the raw water; a sample collection unit that collects the test subject sample from the purified water; and the endotoxin detection device.

An injection water production facility of the disclosure includes: a purified water production unit that obtains purified water from raw water via at least one of a reverse osmosis membrane device that performs reverse osmosis filtration of the raw water or an ion exchange device that performs ion exchange of the raw water; an injection water production unit that obtains injection water from the purified water by a polymer membrane filtration device that filters the purified water or a distiller that distills the purified water; an injection water tank that maintains and stores the injection water in a heated state; a delivery line that delivers the injection water provided in the injection water tank to a predetermined place of use; a sample collection unit that collects the test subject sample from the injection water; and the endotoxin detection device. It is desirable that the sample collection unit collects the sample directly from the injection water tank or from the delivery line on a downstream side of the injection water production unit and on an upstream side of the injection water tank.

The purified water production facility of the disclosure includes treating raw water by at least one of reverse osmosis filtration or ion exchange to obtain purified water, and subjecting the test subject sample collected from the purified water to the method of detecting an endotoxin.

A method of producing an injection water of the disclosure includes treating raw water by at least one of reverse osmosis filtration or ion exchange to obtain purified water, obtaining injection water from the purified water by filtration via polymer membrane filtration of the purified water or by distillation of the purified water, and subjecting the test subject sample collected from the injection water to the method of detecting an endotoxin.

### Advantageous Effects of Invention

As described above, according to the disclosure, it is possible to provide a method of detecting an endotoxin, an endotoxin detection device, a purified water production facility, an injection water production facility, a method of producing purified water and a method of producing injection water that can be easily, quickly (for example, within 30 minutes per detection) and stably quantified even at a low concentration, without utilizing a Limulus test or an electrochemical method.

### BRIEF DESCRIPTION OF DRAWINGS

Fig. 1 is a schematic diagram showing a schematic configuration of a fluorescent substance used in a method of detecting an endotoxin.
Fig. 2 is a schematic diagram showing a configuration of an endotoxin detection device.
Fig. 3 is a schematic diagram showing a schematic configuration of a purified water production facility.
Fig. 4 is a schematic diagram showing a schematic configuration of an injection water production facility.
Fig. 5 is a graph showing a fluorescence spectrum change when an endotoxin has been added to dpa-HCC.
Fig. 6 is a graph showing quantitativity of an endotoxin by dpa-HCC.
Fig. 7 is a graph showing quantitativity of lipid A by dpa-HCC.
Fig. 8 is a graph showing a fluorescence spectrum change when an endotoxin has been added to C1-APB.
Fig. 9 is a graph showing quantitativity of an endotoxin by C1-APB.
Fig. 10 is a graph showing analysis results of an endotoxin by an endotoxin detection device using FIA.
Fig. 11 is a graph showing quantitativity of an endotoxin by an endotoxin detection device using FIA.

### DESCRIPTION OF EMBODIMENTS

Hereinafter, embodiments will be described with reference to the drawings. Note that the drawings are outline diagrams or schematic diagrams, and do not necessarily coincide with actual dimensional ratios.

### < Method of Detecting an Endotoxin >

The method of detecting an endotoxin of the present disclosure is a method of detecting an endotoxin using a fluorescent substance having a structure in which a fluorescent site and a recognition site are connected by a spacer. The recognition site in the fluorescent substance recognizes a specific site of a molecular structure of an endotoxin.

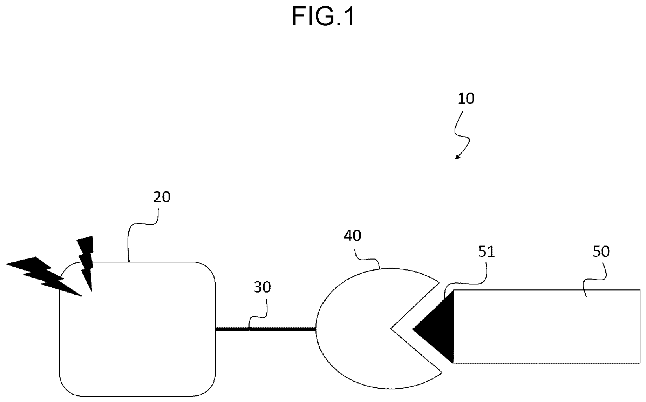

A fluorescent substance 10 has a schematic configuration as shown in the schematic diagram of Fig. 1. That is, a recognition site 40 that recognizes endotoxin 50 and a fluorescent site 20 that emits light are connected by a spacer 30. Metal ions may be coordinated to the recognition site 40 depending on the chemical structure.

The endotoxin 50 has a structure in which a sugar chain that is a hydrophilic site and an acyl acid that is a hydrophobic site are bonded via two molecules of glucosamine. Phosphoric acid is bonded to each glucosamine (Kenichi Tanamoto, "Endotoxin and management of quality of medicine", Bull. Natl. Inst. Health Sci., 126, 19-33 (2008)). Of the endotoxin 50, a portion to which two glucosamines having an acyl acid bonded thereto are bonded is referred to as lipid A, and most of the biological activity of the endotoxin 50 is derived from this portion.

The recognition site 40 recognizes a specific site of the endotoxin 50 according to its molecular structure. In other words, the recognition site 40 is a functional group that physically or chemically interacts with a predetermined site or functional group of the endotoxin 50, and more preferably, among functional groups that specifically interact with a specific site of the endotoxin 50, when recognized, information (hereinafter, it is referred to as "recognition information".) thereof can be transmitted to the fluorescent site 20. The recognition information mentioned here is, for example, a change in the coordination state of electrons inside the chemical structure of the recognition site 40.

In a case in which the recognition site 40 has a chemical structure capable of forming a metal complex, it is desirable to add a metal ion capable of forming a complex that physically or chemically interacts with a functional group constituting the recognition site 40 and a predetermined site or functional group of an endotoxin. In a case in which a mechanism of light emission and quenching of the fluorescent site 20 to be described later depends on photo-induced electron transfer (PET), it is desirable to coordinate a transition metal ion (for example, a copper ion (Cu²⁺), a nickel ion (Ni²⁺), or a cobalt ion (Co²⁺)) whose d orbital is not closed as the metal ion to the recognition site 40. Thereby, since the fluorescent site is quenched when the endotoxin 50 is not present, the detection sensitivity can be enhanced together with S/N ratio.

In a case in which the complex is coordinated, the amount of emission of the fluorescent site 20 significantly increases, so that an endotoxin can be detected as a decrease in the amount of emission in some cases. Examples of such metal ions include zinc ions (Zn²⁺) and cadmium ions (Cd²⁺).

For example, when a chemical structure in which a metal ion Mⁿ⁺, wherein n is a natural number, is coordinated to a dipicolylamino group represented by the following Formula (3) is the recognition site 40 and this metal ion is a copper ion, a nickel ion or a cobalt ion, a zinc ion or a cadmium ion, this recognition site 40 recognizes phosphoric acid bound to glucosamine of the endotoxin 50.

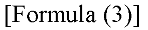

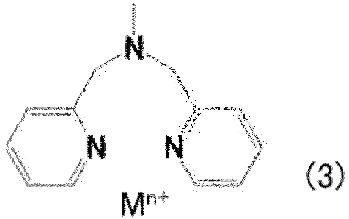

In a case in which phenylboronic acid represented by the following Formula (4) becomes the recognition site 40, this recognition site 40 recognizes the sugar chain of an endotoxin.

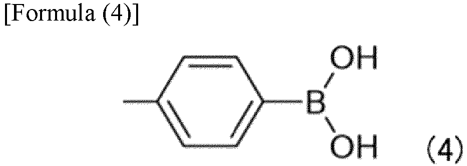

In addition, examples of the chemical structure that can be the recognition site 40 include iminodiacetic acid and a guanidino group.

When the recognition site 40 recognizes the specific site, the coordination state of electrons changes in the chemical structure. This change in the coordination state of the electrons is transmitted to the fluorescent site 20 described later as recognition information. In the fluorescent site 20 to which the recognition information has been transmitted, the coordination state of electrons changes, thereby changing (increasing or attenuating) the emission intensity of the fluorescent site 20, whereby it is visualized that the recognition site 40 recognizes a specific site.

The fluorescent site 20 is a substance having a property of emitting light, and the light emission characteristic changes by receiving recognition information at the recognition site 40. Here, the "emitting light" refers to generating fluorescence or phosphorescence. From the viewpoint of improving the detection sensitivity of an endotoxin by obtaining a large amount of light, generation of fluorescence is more preferable. As such a substance, a substance having a conjugated multiple bond is preferable, and a part of the structure is preferably cyclic. The site of the conjugated multiple bond may be a complex compound containing a substance other than a carbon atom. For example, coumarin represented by the following Formula (5), biphenyl represented by the following Formula (6), pyrene represented by the following Formula (7), stilbene represented by the following Formula (8), anthracene represented by the following Formula (9), and derivatives thereof, an organic electroluminescent dye, and a fluorescent protein can be used as the fluorescent site 20.

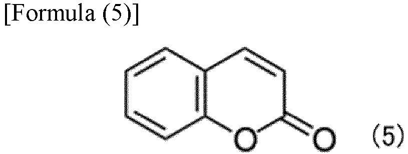

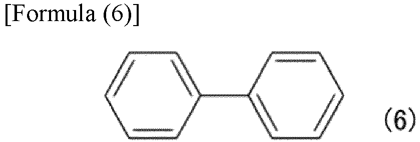

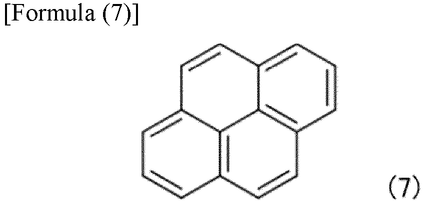

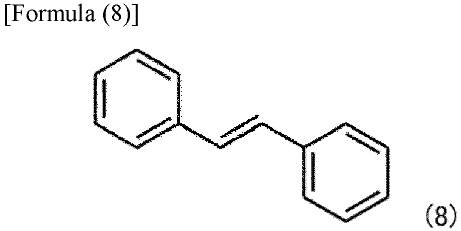

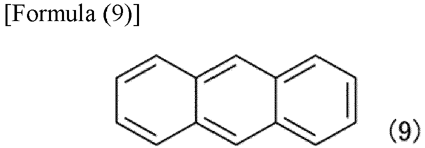

Examples of the coumarin derivative that can be used as the fluorescent site 20 include coumarin 1, coumarin 6, coumarin 7, coumarin 30, 7-hydroxycoumarin-3-carboxylic acid, and 8-(2,2'-dipicolylaminomethyl)-7-hydroxycoumarin-3-carboxylic acid, and 7-hydroxycoumarin-3-carboxylic acid is the most preferable.

Examples of the biphenyl derivative that can be used as the fluorescent site 20 include 4-biphenylcarboxylic acid, 2-(4'-t-butylphenyl)-5-(4'-biphenyl)1,3,4-oxadiazole, 2,5-bis-(2-(4-biphenyl)ethenyl)pyrazine, and 4,4'-bis(2,2-diphenylvinyl)biphenyl, and 4-biphenylcarboxylic acid is the most preferable from the viewpoint of detection sensitivity.

Examples of the pyrene derivative that can be used as the fluorescent site 20 include alkynylpyrene, and the pyrene represented by Formula (7) shown above is the most preferable from the viewpoint of detection sensitivity.

Examples of the stilbene derivative that can be used as the fluorescent site 20 include 1,1,4,4-tetraphenyl-1,3-butadiene, 4,4'-bis(2,2-diphenylvinyl)biphenyl, and cyanostilbene, and the stilbene represented by Formula (8) shown above is the most preferable from the viewpoint of detection sensitivity.

Examples of the anthracene derivative that can be used as the fluorescent site 20 include 10-[2-(9-anthracenyl)ethyl]phenoxazine, 10-[2-(9-anthracenyl)ethyl]phenothiazine, and 2-(9-anthracenyl)ethyldiphenylamine, and the anthracene represented by Formula (9) shown above is the most preferable from the viewpoint of detection sensitivity.

Examples of the fluorescent protein that can be used as the fluorescent site 20 include GFP (Green Fluorescent Protein), BFP (Blue Fluorescent Protein), CFP (Cyan Fluorescent Protein), EGFP (Enhanced Green Fluorescent Protein), EYFP (Enhanced Yellow Fluorescent Protein), and PA-GFP (Photoactivatable Green Fluorescent Protein), and GFP is the most preferable.

The spacer 30 refers to a substance that mediates binding between the fluorescent site 20 and the recognition site 40. More preferably, the substance is a substance having a size and a structure in which the recognition information is efficiently transferred from the recognition site 40 to the fluorescent site 20. Specifically, a linear alkyl chain having from 1 to 10 carbon atoms, a linear alkyl chain in which an atom other than carbon (for example, oxygen, nitrogen or sulfur) is interposed in the middle of the linear alkyl chain, or a derivative in which a side chain is bonded to the linear alkyl chain is desirable. However, it is desirable that the linear chemical bond connecting the recognition site 40 to the fluorescent site 20 is only a single bond.

The fluorescent site 20 often exhibits hydrophobicity, and the recognition site 40 often exhibits hydrophilicity. Therefore, for example, by making the type of the spacer 30 appropriate and imparting sufficient hydrophilicity to the fluorescent substance 10, the fluorescent substance 10 has water solubility, and it is not necessary to dissolve the fluorescent substance 10 in, for example, an organic solvent or the like for use, and the detection accuracy can be increased. As a method of making the type of the spacer 30 appropriate, for example, a method of adjusting the number of carbon atoms of the spacer 30, a method of introducing a watersoluble side chain, or a method of introducing an element such as nitrogen can be considered. Note that similar adjustment can be performed on the fluorescent site 20 and the recognition site 40.

Specific examples of the fluorescent substance include dpa-HCC represented by Formula (1) shown above in which a dipicolylamino group to which a metal ion is coordinated, represented by Formula (3) shown above, and 7-hydroxycoumarin-3-carboxylic acid which is a derivative of coumarin represented by Formula (5) shown above are bonded via a spacer having one carbon atom.

The 7-hydroxycoumarin-3-carboxylic acid, which is the fluorescent site 20 of dpa-HCC, alone shows a fluorescence intensity peak at 448 nm with respect to an excitation wavelength of, for example, 396 nm. In the dpa-HCC in which a dipicolylamino group is bonded to the 7-hydroxycoumarin-3-carboxylic acid via the spacer 30 (-CH₂-), a non-covalent electron pair of a branched nitrogen atom of the dipicolylamino group is moved to a fluorescent site by PET as an electron donor unit, and fluorescence is partially suppressed. When a copper ion, a nickel ion, or a cobalt ion (hereinafter, referred to as "a copper ion or the like".) is coordinated to the dpa-HCC as a metal ion expressed by Chemical Formula 3 shown above, fluorescence is attenuated by LMCT (Ligand to Metal Charge Transfer) in which electrons flow from 7-hydroxycoumarin-3-carboxylic acid as the fluorescent site 20 to an empty d orbital of a copper ion or the like.

However, in the presence of the endotoxin 50, dpa-HCC restores attenuated fluorescence. That is, it is considered that when a phosphate group of the endotoxin 50 is coordinated to a copper ion or the like coordinated to the dipicolylamino group, a chemical bond between the copper ion or the like and dpa-HCC is weakened, so that the attenuated fluorescence of 7-hydroxycoumarin-3-carboxylic acid is restored. That is, the specific site recognized by the recognition site 40 of dpa-HCC as the fluorescent substance 10 is the phosphate group of the endotoxin 50.

Another example of the specific fluorescent substance is C1-APB represented by Formula (2) shown above in which the phenylboronic acid represented by Formula (4) shown above and the pyrene represented by Formula (7) shown above are linked by an amide bond as the spacer 30.

In a state in which the endotoxin 50 is absent, C1-APB is in a state in which fluorescence is attenuated by PET in which electrons flow into phenylboronic acid from pyrene which is the fluorescent site 20. On the other hand, in the presence of the endotoxin 50, C1-APB emits fluorescence. That is, it is considered that the electron acceptability of phenylboronic acid is reduced by an interaction between phenylboronic acid and a hydroxyl group present in a sugar chain moiety of the endotoxin 50, and fluorescence of pyrene which has been attenuated is restored. That is, the specific site recognized by the recognition site 40 of C1-APB as the fluorescent substance 10 is the sugar chain moiety of the endotoxin 50.

Note that, in the method of detecting an endotoxin of the disclosure, a plurality of fluorescent substances 10 having a recognition site 40 that recognizes different specific sites may be used. Thereby, multipoint recognition of an endotoxin becomes possible, and specificity and detection sensitivity are further increased. For example, a mixture of the above-described dpa-HCC and C1-APB is added to a sample as a fluorescent reagent, and the fluorescence intensity can be detected at two wavelengths. The same effect can be obtained by using the fluorescent substance 10 having a plurality of recognition sites 40 or the fluorescent substance 10 having a plurality of fluorescent sites 20. In this case, as the spacer 30, a spacer having a side chain may be used, or a plurality of spacers 30 may be used.

### <Endotoxin Detection Device>

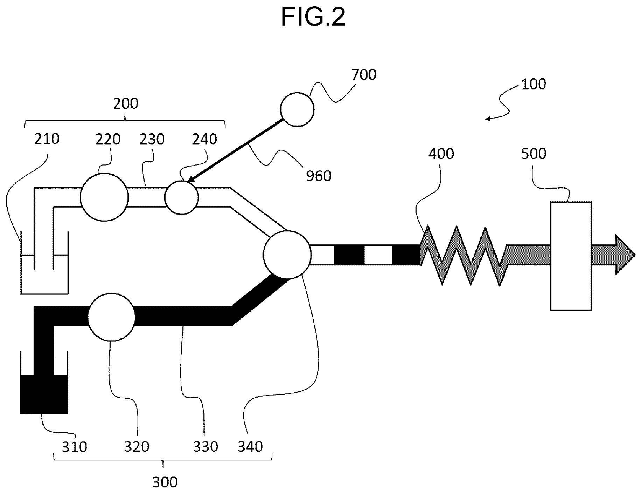

Fig. 2 is a schematic diagram showing an example of an endotoxin detection device 100 of the disclosure. The endotoxin detection device 100 of the disclosure includes a sample introduction unit 200 into which a test subject sample 960 is introduced, a supply unit 300 that supplies the above-described fluorescent substance 10 to the sample, a reaction unit 400 in which the sample 960 and the fluorescent substance 10 react with each other, and a detection unit 500 that detects fluorescence of the fluorescent substance 10 subjected to the reaction. The endotoxin detection device 100 of the present example is configured by using a flow injection analysis method (FIA, Flow Injection Analysis).

The sample introduction unit 200 includes a carrier storage tank 210 that stores a carrier as a liquid that conveys the sample 960, a carrier liquid feed pump 220 that feeds the carrier, a carrier liquid feed path 230 through which the carrier flows, and a sample introduction unit 240 into which the sample 960 is introduced into the carrier liquid feed path 230. The carrier is not particularly limited as long as it is a liquid capable of conveying the sample 960, and for example, pure water or various buffers can be appropriately used according to properties of the sample 960 and the fluorescent substance 10. The sample 960 is a liquid of a test subject for verifying whether the endotoxin 50 is contained. For example, a liquid product such as purified water or injection water as a solvent such as an injection, an infusion solution, or a dialysis fluid, a biological fluid such as blood, saliva, or urine, a diluent thereof, or the like can be the sample 960. It is desirable that the sample 960 collected from the sample collection unit 700 provided in the production line (in particular, purified water production facility and injection water production facility) of the liquid product described above is directly and continuously introduced into the sample introduction unit 240, but the collected sample 960 may be introduced from the sample introduction unit 240 each time of measurement.

The supply unit 300 includes a320
310 that stores a reagent solution containing the fluorescent substance 10, a reagent liquid feed pump 320 that feeds the reagent, a reagent liquid feed path 330 through which the reagent flows, and a mixing unit 340 that joins the carrier liquid feed path 230. In the mixing unit 340, the fluorescent substance 10 is supplied to the sample 960 as a reagent solution and then mixed. As described above, the fluorescent substance 10 has a structure in which the fluorescent site 20 and the recognition site 40 that recognizes a specific site 51 of a molecular structure of the endotoxin 50 are connected by the spacer 30. The meanings of the fluorescent site 20, the recognition site 40, and the spacer 30 are as described above.

In the reaction unit 400, when the endotoxin 50 is contained in the sample 960, a reaction between the fluorescent substance 10 in the reagent and the endotoxin 50 occurs, and light is emitted from the fluorescent substance 10. As the reaction unit 400, a reaction coil adopted in a general detection system is used, but a simple capillary tube may be used as the reaction unit 400.

In the detection unit 500, emission of the fluorescent substance 10 subjected to the above-described reaction is optically detected. As the detection unit 500, a general light emitting detector can be used.

The endotoxin detection device 100 of the disclosure is not limited to the configuration using the above-described FIA, and can also be realized by, for example, a configuration using FCA (liquid flow analysis method) including CFA (continuous flow analysis method), SIA (sequential injection analysis method), and r-FIA (reverse flow injection analysis method).

In the endotoxin detection device 100 of the disclosure, it is desirable to use dpa-HCC (Formula (1) shown above) that reacts with a phosphate group as the specific site 51 or C1-APB (Formula (2) shown above) that reacts with a sugar chain moiety as the specific site 51 as the fluorescent substance 10.

### <Purified Water Production Facility>

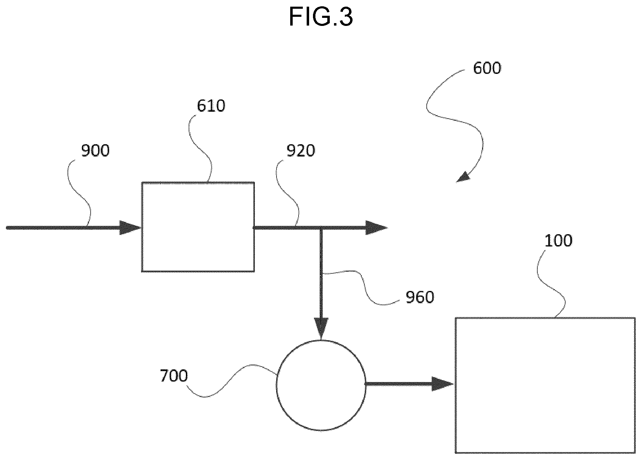

Fig. 3 is a schematic diagram showing a schematic configuration of an example of a purified water production facility 600 of the disclosure. A purified water production facility 600 of the disclosure includes a purified water production unit 610 that obtains purified water 920 from raw water 900, a sample collection unit 700 that collects the sample 960 of a test subject from the purified water 920, and the endotoxin detection device 100.

The purified water production unit 610 is, for example, a facility that removes impurities from the raw water 900 by at least one of a reverse osmosis membrane device that performs reverse osmosis filtration of the raw water 900 or an ion exchange device that performs ion exchange of the raw water to produce the purified water 920. As the ion exchange device, for example, an electric desalting device or a mixed bed ion exchange resin device can be used.

The sample collection unit 700 is provided on a downstream side of the purified water production unit 610 and in the middle of a conveying line of the purified water 920. The purified water 920 collected by the sample collection unit 700 is introduced into the endotoxin detection device 100 from the sample introduction unit 240 (see Fig. 2) as the sample 960 of a test subject. Details of the endotoxin detection device 100 are as described above. As a result, the sample 960 of a test subject collected from the purified water 920 is subjected to the method of detecting an endotoxin described above.

With the above configuration, in the purified water production facility 600 of the disclosure, it is possible to collect the sample 960 from the produced purified water 920 as needed and detect the endotoxin 50 in real time by the endotoxin detection device 100. In a case in which the concentration of the endotoxin 50 exceeds a predetermined reference value, for example, the purified water production facility 600 is immediately stopped, and appropriate measures such as component replacement or repair can be taken.

### <Injection Water Production Facility>

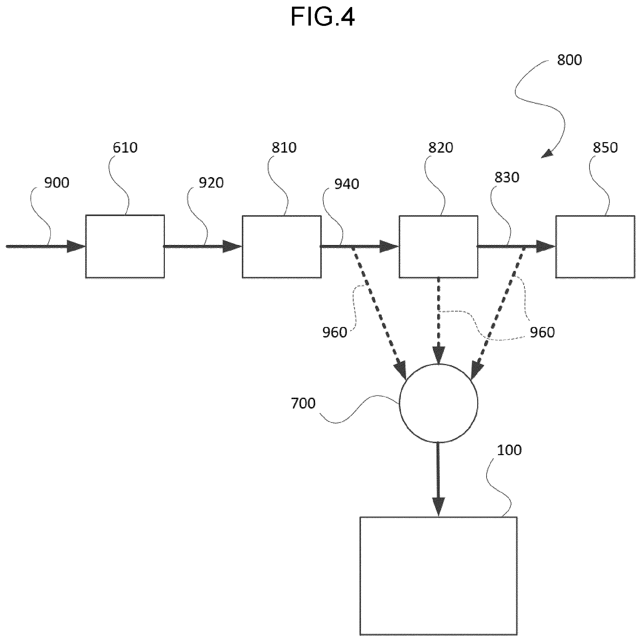

Fig. 4 is a schematic diagram showing a schematic configuration of an example of an injection water production facility 800 of the disclosure. An injection water production facility 800 of the disclosure includes the purified water production unit 610 that obtains purified water 920 from raw water 900, an injection water production unit 810 that obtains injection water 940 from purified water 920, an injection water tank 820 that maintains and stores the injection water 940 in a heated state, a delivery line 830 that delivers the injection water 940 provided in the injection water tank 820 to a predetermined place of use 850, a sample collection unit 700 that collects the test subject sample 960 from the injection water 940, and the endotoxin detection device 100 described above.

The purified water production unit 610 is as described in the purified water production facility 600.

In the injection water production unit 810, the purified water 920 is filtered by a polymer membrane filtration device (preferably an ultrafiltration membrane device) to highly remove impurities such as the endotoxin 50, thereby producing the injection water 940. A distiller may be used instead of the polymer membrane filtration device.

In a case in which the purified water production unit 610 is provided with the endotoxin detection device 100 and the endotoxin concentration of the purified water 920 is known, operating conditions of the injection water production unit 810 may be adjusted accordingly. For example, when it is known that the endotoxin concentration of the purified water 920 is sufficiently low, the endotoxin removal ability may be lowered by that amount to increase the amount of permeated water of the polymer membrane filtration device, and the production amount of the injection water 940 may be increased.

A heating device such as a heat exchanger is attached to the injection water tank 820, and a temperature state of, for example, 60°C or higher, desirably 70°C or higher is maintained in order to prevent growth of bacteria and the like in the injection water 940 and maintain a clean state. In order to prevent retention of the injection water 940 while maintaining this temperature state, it is desirable to form the injection water tank 820 using a circulation pipe.

The delivery line 830 delivers the injection water 940 stored in the injection water tank 820 to the predetermined place of use 850 as needed or if necessary. As the predetermined place of use 850, for example, various facilities according to the use of the injection water 940, such as a packaging facility of the injection water 940 or a production facility of an injection solution, are assumed.

The sample collection unit 700 is provided at a predetermined location on the downstream side of the injection water production unit 810 in the injection water production facility 800. The purified water 920 collected by the sample collection unit 700 is introduced into the endotoxin detection device 100 from the sample introduction unit 240 (see Fig. 2) as the test subject sample 960. Details of the endotoxin detection device 100 are as described above. As shown in Fig. 4, the sample collection unit 700 may be provided at any one of a location on the downstream side of the injection water production unit 810 and on an upstream side of the injection water tank 820, a location directly in the injection water tank 820 (for example, in the middle of the circulation pipe), and a location in the middle of the delivery line 830. In any case, the test subject sample 960 collected from the injection water 940 is subjected to the method of detecting an endotoxin described above.

In a case in which the sample collection unit 700 is provided on the downstream side of the injection water production unit 810 and on the upstream side of the injection water tank 820, it is possible to substantially constantly confirm whether the endotoxin concentration of the injection water 940 immediately after being produced by the injection water production unit 810 satisfies, for example, the standard described in the Japanese Pharmacopoeia (0.25 EU/mL). Then, in a case in which this standard is not satisfied by any chance, it is possible to prevent contamination of the injection water 940 in a state of satisfying the standard stored in the injection water tank 820 on the downstream at that time by immediately stopping supply of the injection water 940 to the downstream. Note that the "substantially constantly" refers to, for example, measurement in which the repeated measurement time is within 30 minutes, preferably within 10 minutes. In consideration of the amount of water held by the device on the downstream side and the amount of water produced by the device, by repeating the measurement at these frequencies, it is possible to operate without stopping the production of injection water due to contamination due to water quality deterioration due to trouble.

In a case in which the sample collection unit 700 is directly provided in the injection water tank 820, it is possible to substantially constantly monitor whether the circulated and stored injection water 940 satisfies the above standard. Then, for example, the injection water 940 may be supplied to the delivery line 830 only when it is confirmed that the above standard is satisfied, and when the above standard is not satisfied, the injection water 940 may be returned to the upstream side of the injection water production unit 810 using a return line (not shown) and reprocessed in the injection water production unit 810.

Since the endotoxin detection device 100 of the invention has sufficient measurement accuracy, it is possible to confirm not only a defect of the device at the preceding stage but also a sign of the defect by substantially constantly monitoring. For example, in a case in which the endotoxin measured value of the injection water 940 to be produced satisfies the standard value of an endotoxin but shows a slight rising tendency, this is often a sign of deterioration of the performance of the device at the preceding stage, and when this is left as it is, the injection water 940 to be produced may not satisfy the standard value of an endotoxin. That is, the trouble can be avoided by detecting the defect of the injection water production device in advance. For example, it is possible to cope with the defect by changing the operating condition of the device at the preceding stage or performing maintenance.

In a case in which the sample collection unit 700 is provided in the delivery line 830, it is possible to directly monitor the endotoxin concentration of the injection water 940 at the time when the injection water is actually used. For example, it is also conceivable to use a batch type in which a tank is installed immediately before the place of use 850, injection water is stored in the tank, the injection water is supplied to the place of use 850, and the injection water is stored again when the tank becomes empty. Then, by confirming the endotoxin concentration of the injection water in the tank with the endotoxin detection device 100 for each batch, it is possible to guarantee the use of injection water whose endotoxin concentration satisfies the management standard.

All of the three locations described above have an advantage of installing the sample collection unit 700, and are preferable installation locations. However, it is the most preferable to provide the sample collection unit 700 on the downstream side of the injection water production unit 810 and on the upstream side of the injection water tank 820 in that contamination of the injection water 940 with the endotoxin 50 can be detected at the earliest. If possible, the sample collection units 700 may be installed at two or more of the three locations.

With the above configuration, in the injection water production facility 800 of the disclosure, it is possible to collect the sample 960 from the produced injection water 940 as needed and detect the endotoxin 50 in real time by the endotoxin detection device 100. Then, in a case in which the concentration of the endotoxin 50 exceeds a predetermined reference value, the injection water production facility 800 is immediately stopped, and appropriate measures such as component replacement or repair can be taken. Thereby, the injection water 940 with the required water quality can be stably manufactured.

In the injection water production facility 800 of the disclosure, the impurity concentration of the injection water 940 to be the sample 960 is sufficiently small, and the influence of other impurities can be minimized, so that the endotoxin concentration can be particularly accurately measured. Since the viscosity of the injection water 940 to be the sample 960 is sufficiently low, it is not particularly necessary to dilute the injection water 940 with a carrier solution or the like, and it is possible to directly deliver the injection water 940 to the endotoxin detection device 100. This also has an advantage that the carrier solution is not required and the sample 960 is not unnecessarily diluted with the carrier.

In a case in which the sample collection unit 700 is installed directly in the injection water tank 820 or in the delivery line 830, the sample 960 is collected in a heated state. The sample 960 with such a high temperature is preferably cooled to room temperature by a heat exchanger or the like before being supplied to the endotoxin detection device 100.

### Examples

### (1) Measurement of an Endotoxin by dpa-HCC

Measurement of an endotoxin was attempted using dpa-HCC. The experimental method was as follows.

As measurement samples, Sample 1 and Sample 2 with compositions shown in Table 1 below were prepared in a 10 mL volumetric flask.

**[Table 1]**

| Component | Sample | |
|---|---|---|
| | 1 | 2 |
| HEPES buffer (pH 7.4) | 5 mM | |
| NaNO₃ | 100 mM | |
| Cu (NO₃)₂ | 0.01 mM | |
| dpa-HCC | 0.01 mM | |
| Endotoxin | 0.0 EU/mL | 130 EU/mL |

Sample 1 and Sample 2 were taken in a quartz cell with an optical path length of 1 cm, and a fluorescence spectrum was measured with a fluorescence spectrophotometer. The measurement conditions were as follows.
Excitation wavelength: 358 nm
Start wavelength: 380 nm
End wavelength: 600 nm

According to the measurement conditions, measurement of each sample could be completed within 5 minutes.

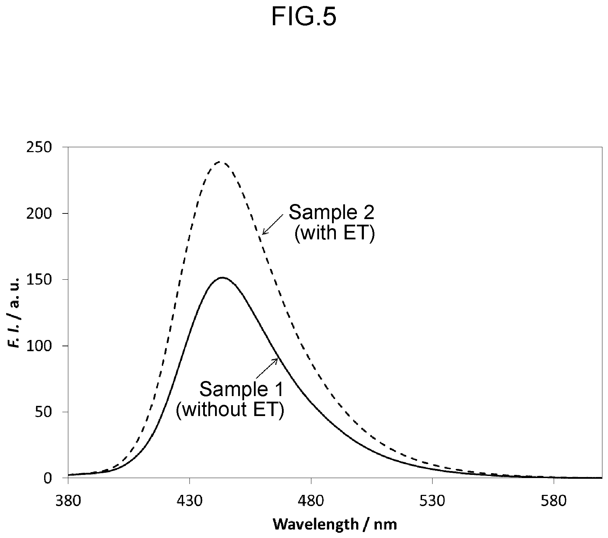

Fluorescence spectra of Sample 1 (solid line) and Sample 2 (broken line) are as shown in Fig. 5. In Fig. 5, the vertical axis represents fluorescence intensity, and the horizontal axis represents wavelength (nm), respectively. Both Sample 1 and Sample 2 showed a peak of fluorescence intensity at a wavelength of 443 nm. Then, Sample 2 (broken line) to which an endotoxin (Endotoxin is denoted as ET in the figure. The same applies to the following figures.) had been added showed a fluorescence intensity about 1.6 times that of Sample 1 (solid line) to which an endotoxin had not been added. From the above, it was shown that an endotoxin enhanced the fluorescence of dpa-HCC coordinated with copper ions. 7-Hydroxycoumarin-3-carboxylic acid, which is a fluorescent site of dpa-HCC, alone shows a fluorescence intensity peak at 448 nm with respect to an excitation wavelength of, for example, 396 nm. However, in a case where an endotoxin is measured using dpa-HCC, in a case in which the intensity of the peak at a wavelength of 443 nm was measured with an excitation wavelength of 358 nm, quantitativity and the like were better.

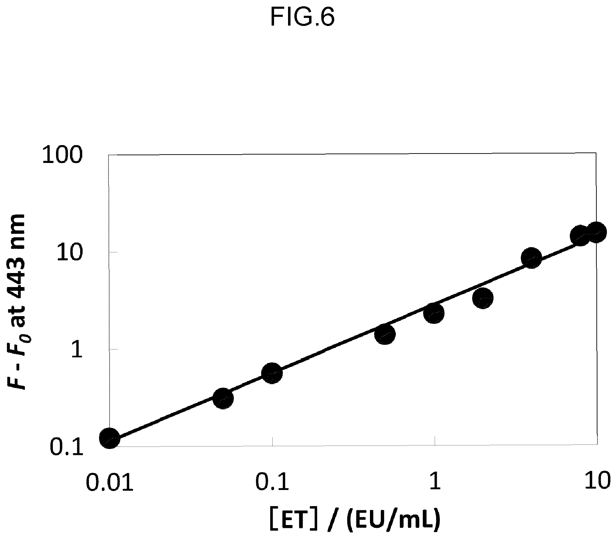

Fig. 6 is a graph showing measurement results of each sample in which the endotoxin concentration was changed to 0.01 to 10 EU in the composition of Sample 2. For sample preparation, a control standard endotoxin (293-16541 (derived from E. Coli UKTB strain) manufactured by FUJIFILM Wako Pure Chemical Corporation) was used. In Fig. 6, the horizontal axis represents the logarithm of the endotoxin concentration (EU/mL), and the vertical axis represents the logarithm of the fluorescence spectrum change (value obtained by subtracting fluorescence intensity (F₀) at a wavelength of 443 nm of Sample 1 to which an endotoxin had not been added from fluorescence intensity (F) at a wavelength of 443 nm of the sample to which an endotoxin had been added). From this graph, it is inferred that the logarithm of the fluorescence intensity at a wavelength of 443 nm has a strong positive correlation with the logarithm of the endotoxin concentration. Therefore, it was considered that endotoxin at a low concentration of from 0.01 to 10 EU can be measured by dpa-HCC coordinated with copper ions.

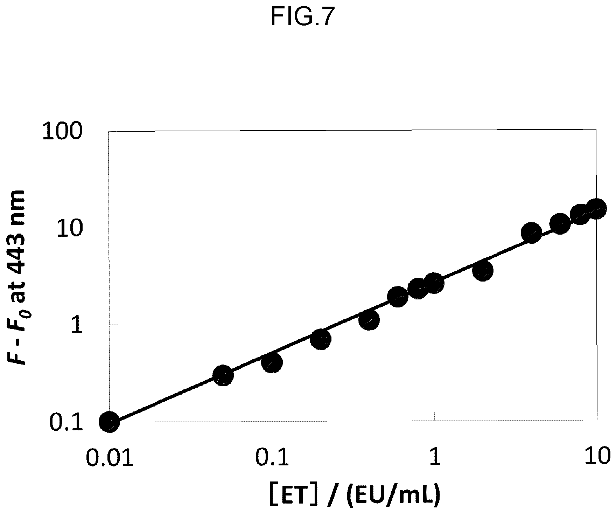

Fig. 7 is a graph showing measurement results of each sample in which the concentration of lipid A was changed to 0.01 to 10 EU in place of an endotoxin. The horizontal axis and the vertical axis in Fig. 7 are similar to those in Fig. 6. Here, since the titer of a 10 pM endotoxin generally corresponds to 1 EU/mL, 10 pM lipid A is similarly converted to 1 EU/mL. From this graph, it is inferred that the logarithm of the fluorescence intensity at a wavelength of 443 nm has a strong positive correlation with the logarithm of the lipid A concentration. As described above, the graph obtained by measuring an endotoxin in Fig. 6 showed almost the same behavior as the graph of lipid A, which is considered to be the center of the biological activity of an endotoxin, and thus it was inferred that the measurement of an endotoxin in Fig. 6 depends on the recognition of the phosphate group that binds to glucosamine of an endotoxin by dpa-HCC coordinated with copper ions.

Various phosphate anions were added instead of an endotoxin in the same procedure as described above, and results of measuring the fluorescence spectrum change are shown in the following Table 2. The endotoxin concentration was set to 1.3 nM, and the other phosphate anion concentrations were set to 1.0 mM. Here, since the titer of a 100 pg endotoxin generally corresponds to 1 EU, 100 EU/mL is converted to 1 nM.

**[Table 2]**

| Anion phosphate | Fluorescence intensity per unit molar concentration ((F - F₀)/nM) |
|---|---|
| Endotoxin | 68 |
| Phosphoric acid | 1.4 × 10⁻⁵ |
| Pyrophosphoric acid | 8.7 × 10⁻⁶ |
| Triphosphoric acid | -6.3 × 10⁻⁶ |
| AMP | 1.7 × 10⁻⁵ |
| ADP | 1.5 × 10⁻⁵ |
| ATP | -1.2 × 10⁻⁵ |

From the above results, it was presumed that dpa-HCC coordinated with copper ions has the highest selectivity for an endotoxin even among the same phosphate compounds, and recognizes lipid A, which is the bioactive center of an endotoxin.

### (2) Measurement of an Endotoxin by C1-APB

Measurement of an endotoxin was attempted using C1-APB. The experimental method was as follows.

As measurement samples, Sample 3 and Sample 4 with compositions shown in Table 3 below were prepared in a 10 mL volumetric flask.

**[Table 3]**

| Component | Sample | |
|---|---|---|
| | 3 | 4 |
| Phosphoric acid buffer (pH 7.4) | 10 mM | |
| NaCl | 100 mM | |
| C1-APB DMSO solution | 0.01 mM | |
| Endotoxin | 0.0 EU/mL | 130 EU/mL |

Sample 3 and Sample 4 were taken in a quartz cell with an optical path length of 1 cm, and a fluorescence spectrum was measured with a fluorescence spectrophotometer. The measurement conditions were as follows.
Excitation wavelength: 328 nm
Start wavelength: 350 nm
End wavelength: 500 nm

According to the measurement conditions, measurement of each sample could be completed within 5 minutes.

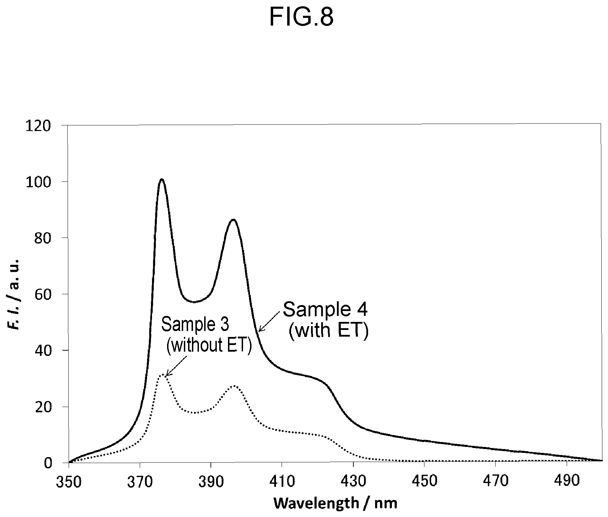

Fluorescence spectra of Sample 3 and Sample 4 are as shown in Fig. 8. In Fig. 8, the vertical axis represents fluorescence intensity, and the horizontal axis represents wavelength (nm), respectively. Both Sample 3 and Sample 4 showed fluorescence intensity peaks at wavelengths of 376 nm and 396 nm. Then, Sample 4 (solid line) to which an endotoxin had been added showed a fluorescence intensity about three times that of Sample 3 (dotted line) to which an endotoxin had not been added. From the above, it was shown that an endotoxin enhanced the fluorescence of C1-APB.

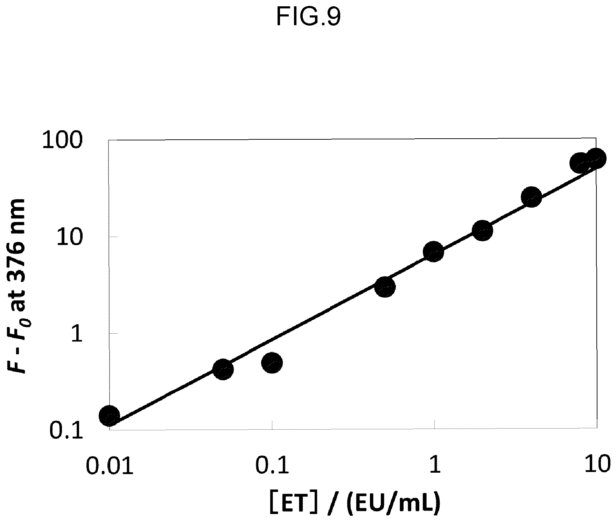

Fig. 9 is a graph showing measurement results of each sample in which the endotoxin concentration was changed to 0.01 to 10 EU in the composition of Sample 4. In Fig. 9, the horizontal axis represents the logarithm of the endotoxin concentration (EU/mL), and the vertical axis represents the logarithm of the fluorescence spectrum change (value obtained by subtracting fluorescence intensity (F₀) at a wavelength of 376 nm of Sample 3 to which an endotoxin had not been added from fluorescence intensity (F) at a wavelength of 376 nm of the sample to which an endotoxin had been added). From this graph, it is inferred that the logarithm of the fluorescence intensity at a wavelength of 376 nm has a strong positive correlation with the logarithm of the endotoxin concentration. Therefore, it was considered that an endotoxin at a low concentration of from 0.01 to 10 EU can be measured by C1-APB.

In order to confirm that C1-APB specifically recognizes the sugar chain of an endotoxin, various kinds of sugars were added instead of an endotoxin, and results of measuring the fluorescence spectrum change are shown in the following Table 4. The endotoxin concentration was set to 1.3 nM, and the other sugar concentrations were set to 30 mM. Here, since the titer of a 100 pg endotoxin generally corresponds to 1 EU, 100 EU/mL is converted to 1 nM.

**[Table 4]**

| Sugar | Fluorescence intensity per unit molar concentration ((F - F₀)/nM) |
|---|---|
| Endotoxin | 50 |
| Lactose | 1.0 × 10⁻⁷ |
| Galactose | 3.0 × 10⁻⁷ |
| Glucose | 7.7 × 10⁻⁷ |
| Fructose | 1.7 × 10⁻⁶ |
| Mannose | 6.7 × 10⁻⁷ |

From the above, it was presumed that C1-APB has the highest selectivity for an endotoxin even among the same sugar compounds.

### (3) Quantitativity of an Endotoxin by FIA

Quantitativity of an endotoxin by an endotoxin detection device using FIA as shown in Fig. 2 was verified. A specific verification method is as follows.

Ultrapure water was used as a carrier. dpa-HCC was used as the fluorescent substance, and a reagent was prepared with the following composition, and then pH was adjusted to 7.4.
dpa-HCC: 0.02 mM
NaNO₃: 200 mM
HEPES: 10 mM
Cu(NO₃)₂: 0.02 mM

As samples containing an endotoxin as test subjects, aqueous solutions obtained by diluting a standard endotoxin to 0.2 EU/mL, 2 EU/mL, 20 EU/mL, and 200 EU/mL were prepared. The samples were added to the carrier by a syringe from the sample introduction unit.

The measurement conditions were as follows.
Carrier flow rate: 0.5 mL/min
Reagent flow rate: 0.5 mL/min
Excitation wavelength: 358 nm
Fluorescence wavelength: 443 nm

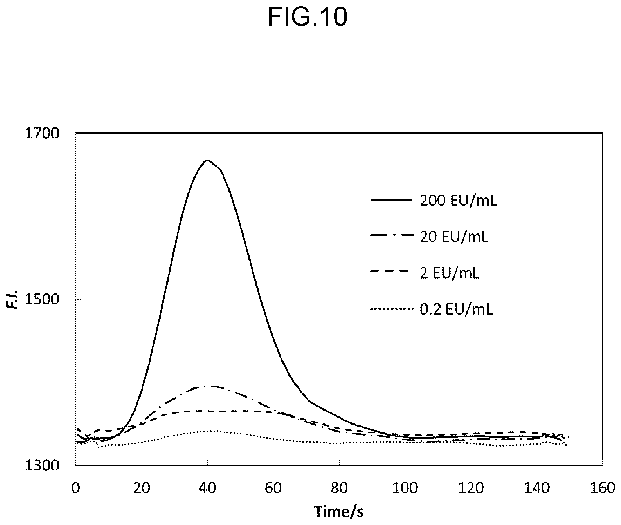

The measurement results are shown in Fig. 10. In Fig. 10, the vertical axis represents fluorescence intensity, and the horizontal axis represents time (s) after sample addition. The fluorescence intensity peaked at 40 seconds after addition of the sample at any endotoxin concentration. From this result, it is inferred that the endotoxin concentration over time can be measured by an endotoxin detection device using FIA.

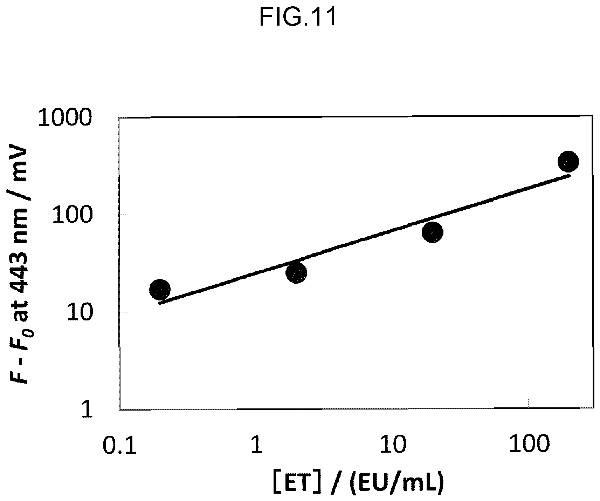

At this peak time shown in Fig. 10, as shown in the graph of Fig. 11 in which the relationship between the endotoxin concentration and the fluorescence spectrum change is plotted (the vertical axis and the horizontal axis are the same as those in Figs. 6 and 7), it is inferred that there is a strong positive correlation between the logarithm of the endotoxin concentration and the fluorescence intensity. As a conclusion, it was inferred that quantitative measurement of an endotoxin over time can be performed by an endotoxin detection device using FIA. It has become possible to quickly measure one sample by online within 3 minutes. That is, it could be confirmed that the samples with an endotoxin concentration of from 0.2 EU/mL to 200 EU/mL used in this example can be measured by the measurement conditions selected in this example.

According to the method of detecting an endotoxin of the disclosure, by appropriately selecting measurement conditions such as an excitation wavelength, a fluorescence wavelength, an amount of a fluorescent substance, and a reaction time according to properties of a sample or the like, it is possible to quantify from 0.01 EU/mL to 10 EU/mL, which is a concentration sufficient to prophylactically manage less than 0.25 EU/mL which is a management standard for an endotoxin in injection water. It is possible to measure an endotoxin concentration of 0.01 EU/mL or more within 5 minutes, and depending on conditions, within 3 minutes.

For example, a sample concentration device may be installed immediately before the sample introduction unit of the endotoxin detection device if necessary, and the sample may be introduced after being concentrated to a target magnification. This makes it possible to evaluate a sample with a lower endotoxin concentration. As the sample concentration device, for example, an ultrafiltration membrane device (UF) or a reverse osmosis membrane device (RO) can be used.

### Industrial Application Field

The present disclosure can be used for an endotoxin detection device, a purified water production facility, and an injection water production facility.

The present disclosure comprises the following aspects:
Aspect 1: A method of detecting an endotoxin, the method comprising:
   detecting an endotoxin from a test subject sample using a fluorescent substance having a structure in which a fluorescent site and a recognition site are connected by a spacer,
   wherein the recognition site recognizes a specific site of a molecular structure of an endotoxin.
Aspect 2: The method of detecting an endotoxin according to aspect 1, wherein the spacer in the fluorescent substance has from 1 to 10 carbon atoms, and each chemical bond of a straight chain connecting the fluorescent site to the recognition site via the spacer is only a single bond.
Aspect 3: The method of detecting an endotoxin according to aspect 2, wherein the fluorescent substance is dpa-HCC represented by the following Formula 1, which has a dipicolylamino group as the recognition site to which a metal ion Mⁿ⁺, wherein n is a natural number, is coordinated, and has 7-hydroxycoumarin-3-carboxylic acid as the fluorescent site, and wherein the spacer has one carbon atom, and the specific site is a phosphate group of an endotoxin: 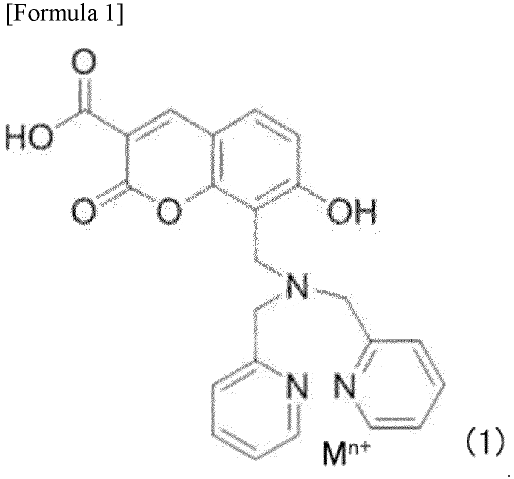
Aspect 4: The method of detecting an endotoxin according to aspect 3, wherein the metal ion is a copper ion (Cu²⁺), a nickel ion (Ni²⁺), or a cobalt ion (Co²⁺).
Aspect 5: The method of detecting an endotoxin according to aspect 2, wherein the fluorescent substance is C1-APB represented by the following Formula 2, which has phenylboronic acid as the recognition site, and has pyrene as the fluorescent site, and wherein the spacer has an amide bond, and the specific site is a sugar chain moiety of an endotoxin: 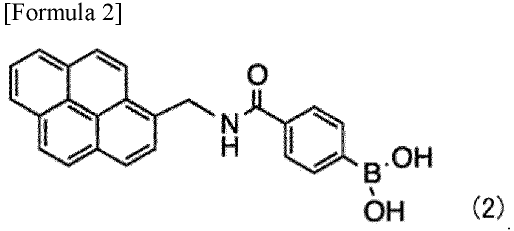
Aspect 6: An endotoxin detection device, comprising:
   a sample introduction unit into which a test subject sample is introduced;
   a supply unit configured to supply a fluorescent substance to the sample, the fluorescent substance having a structure in which a fluorescent site and a recognition site that recognizes a specific site of a molecular structure of an endotoxin are connected by a spacer;
   a reaction unit in which the sample and the fluorescent substance react with each other; and
   a detection unit configured to detect light emission or color development of the fluorescent substance subjected to the reaction.
Aspect 7: The endotoxin detection device according to aspect 6, wherein the spacer in the fluorescent substance has from 1 to 10 carbon atoms, and each chemical bond of a straight chain connecting the fluorescent site to the recognition site via the spacer is only a single bond.
Aspect 8: The endotoxin detection device according to aspect 7, wherein the fluorescent substance is dpa-HCC represented by the following Formula 3, which has a dipicolylamino group as the recognition site to which a metal ion Mⁿ⁺, wherein n is a natural number, is coordinated, and has 7-hydroxycoumarin-3-carboxylic acid as the fluorescent site, and wherein the spacer has one carbon atom, and the specific site is a phosphate group of an endotoxin: 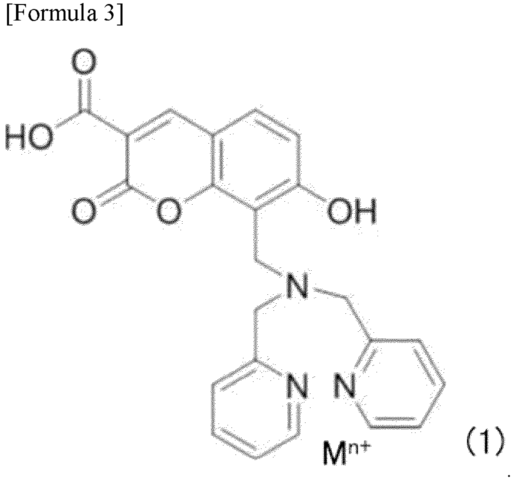
Aspect 9: The endotoxin detection device according to aspect 8, wherein the metal ion is a copper ion (Cu²⁺), a nickel ion (Ni²⁺), or a cobalt ion (Co²⁺).
Aspect 10: The endotoxin detection device according to aspect 7, wherein the fluorescent substance is C1-APB represented by the following Formula 4, which has phenylboronic acid as the recognition site, and has pyrene as the fluorescent site, and wherein the spacer has an amide bond, and the specific site is a sugar chain moiety of an endotoxin: 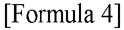 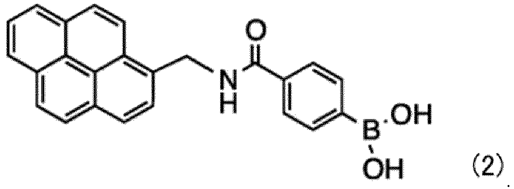
Aspect 11: The endotoxin detection device according to any one of aspects 6 to 10, wherein the sample is collected from purified water produced in a purified water production facility or injection water produced in an injection water production facility.
Aspect 12: A purified water production facility, comprising:
   a purified water production unit that obtains purified water from raw water via at least one of a reverse osmosis membrane device that performs reverse osmosis filtration of the raw water or an ion exchange device that performs ion exchange of the raw water;
   a sample collection unit that collects the test subject sample from the purified water; and
   the endotoxin detection device according to any one of aspects 6 to 11.
Aspect 13: An injection water production facility, comprising:
   a purified water production unit that obtains purified water from raw water via at least one of a reverse osmosis membrane device that performs reverse osmosis filtration of the raw water or an ion exchange device that performs ion exchange of the raw water;
   an injection water production unit that obtains injection water from the purified water via a polymer membrane filtration device that filters the purified water or a distiller that distills the purified water;
   an injection water tank that maintains and stores the injection water in a heated state;
   a delivery line that delivers the injection water provided in the injection water tank to a predetermined place of use;
   a sample collection unit that collects the test subject sample from the injection water; and
   the endotoxin detection device according to any one of aspects 6 to 11.
Aspect 14: The injection water production facility according to aspect 13, wherein the sample collection unit collects the sample directly from the injection water tank or from the delivery line at a downstream side of the injection water production unit and at an upstream side of the injection water tank.
Aspect 15: A method of producing purified water, the method comprising:
   treating raw water by at least one of reverse osmosis filtration or ion exchange to obtain purified water; and
   subjecting a test subject sample collected from the purified water to the method of detecting an endotoxin according to any one of aspects 1 to 5.
Aspect 16: A method of producing injection water, the method comprising:
   treating raw water by at least one of reverse osmosis filtration or ion exchange to obtain purified water;
   obtaining injection water from the purified water by filtration via polymer membrane filtration of the purified water or by distillation of the purified water; and
   subjecting a test subject sample collected from the injection water to the method of detecting an endotoxin according to any one of aspects 1 to 5.

## Claims

1. An injection water production facility (800), comprising:
a purified water production unit (610) that obtains purified water (920) from raw water (900) via at least one of a reverse osmosis membrane device that performs reverse osmosis filtration of the raw water (900) or an ion exchange device that performs ion exchange of the raw water (900);
an injection water production unit (810) that obtains injection water (940) from the purified water (920) via a polymer membrane filtration device that filters the purified water (920) or a distiller that distills the purified water (920);
an injection water tank (820) that maintains and stores the injection water (940) in a heated state;
a delivery line (830) that delivers the injection water (940) provided in the injection water tank (820) to a predetermined place of use (850);
a sample collection unit (700) that collects a test subject sample (960) from the injection water (940); and
an endotoxin detection device (100) comprising
a sample introduction unit (200) into which the test subject sample (960) is introduced;
a supply unit (300) configured to supply a fluorescent substance (10) to the sample (960), the fluorescent substance (10) having a structure in which a fluorescent site (20) and a recognition site (40) that recognizes a specific site (51) of a molecular structure of an endotoxin (50) are connected by a spacer (30);
a reaction unit (400) in which the sample (960) and the fluorescent substance (10) react with each other; and
a detection unit (500) configured to detect light emission or color development of the fluorescent substance (10) subjected to the reaction.

2. The injection water production facility (800) according to claim 1, wherein the sample collection unit (700) collects the sample (960) directly from the injection water tank (820) or from the delivery line (830) at a downstream side of the injection water production unit (810) and at an upstream side of the injection water tank (820).

3. The injection water production facility (800) according to claim 1 or claim 2, wherein the spacer (30) in the fluorescent substance (10) has from 1 to 10 carbon atoms, and each chemical bond of a straight chain connecting the fluorescent site (20) to the recognition site (40) via the spacer (30) is only a single bond.

4. The injection water production facility (800) according to claim 3, wherein the fluorescent substance (10) is dpa-HCC represented by the following Formula 3, which has a dipicolylamino group as the recognition site (40) to which a metal ion Mⁿ⁺, wherein n is a natural number, is coordinated, and has 7-hydroxycoumarin-3-carboxylic acid as the fluorescent site (20), and wherein the spacer (30) has one carbon atom, and the specific site (51) is a phosphate group of an endotoxin (50):

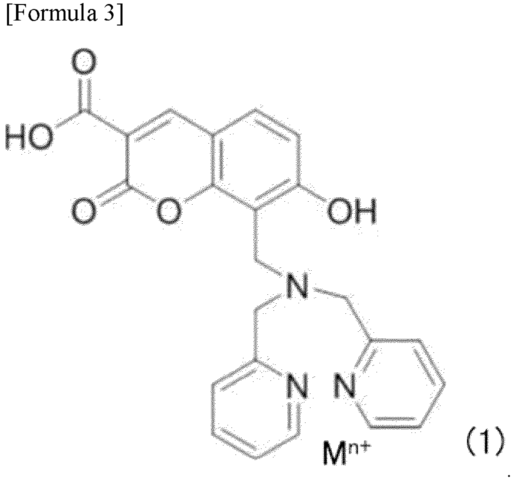

5. The injection water production facility (800) according to claim 4, wherein the metal ion is a copper ion (Cu²⁺), a nickel ion (Ni²⁺), or a cobalt ion (Co²⁺).

6. The injection water production facility (800) according to claim 3, wherein the fluorescent substance (10) is C1-APB represented by the following Formula 4, which has phenylboronic acid as the recognition site (40), and has pyrene as the fluorescent site (20), and wherein the spacer (30) has an amide bond, and the specific site (51) is a sugar chain moiety of an endotoxin (50):

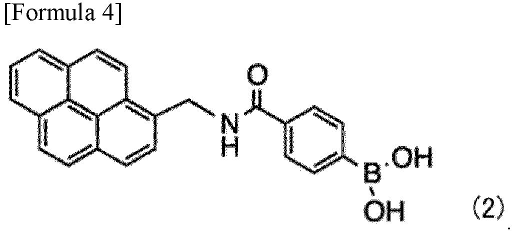

7. A method of producing injection water, the method comprising:
treating raw water (900) by at least one of reverse osmosis filtration or ion exchange to obtain purified water (920);
obtaining injection water (940) from the purified water (920) by filtration via polymer membrane filtration of the purified water (920) or by distillation of the purified water (920); and
subjecting a test subject sample (960) collected from the injection water (940) to a method of detecting an endotoxin, the method comprising:
detecting an endotoxin (50) from the test subject sample (960) using a fluorescent substance (10) having a structure in which a fluorescent site (20) and a recognition site (40) are connected by a spacer (30),
wherein the recognition site (40) recognizes a specific site (51) of a molecular structure of an endotoxin (50).

8. The method of producing injection water according to claim 7, wherein the spacer (30) in the fluorescent substance (10) has from 1 to 10 carbon atoms, and each chemical bond of a straight chain connecting the fluorescent site (20) to the recognition site (40) via the spacer (30) is only a single bond.

9. The method of producing injection water according to claim 8, wherein the fluorescent substance (10) is dpa-HCC represented by the following Formula 1, which has a dipicolylamino group as the recognition site (40) to which a metal ion Mⁿ⁺, wherein n is a natural number, is coordinated, and has 7-hydroxycoumarin-3-carboxylic acid as the fluorescent site (20), and wherein the spacer (30) has one carbon atom, and the specific site (51) is a phosphate group of an endotoxin (50):

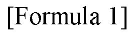

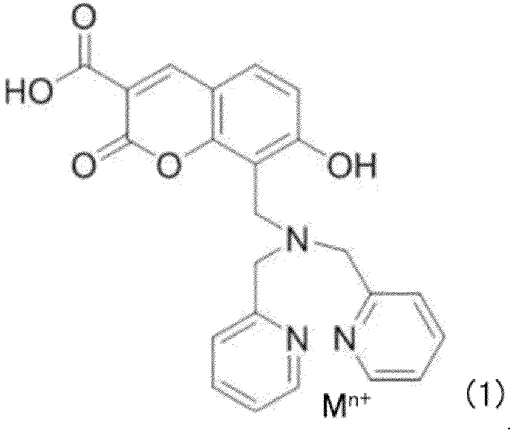

10. The method of producing injection water according to claim 9, wherein the metal ion is a copper ion (Cu²⁺), a nickel ion (Ni²⁺), or a cobalt ion (Co²⁺).

11. The method of producing injection water according to claim 8, wherein the fluorescent substance (10) is C1-APB represented by the following Formula 2, which has phenylboronic acid as the recognition site (40), and has pyrene as the fluorescent site (20), and wherein the spacer (30) has an amide bond, and the specific site (51) is a sugar chain moiety of an endotoxin (50):
